# EUROPÄISCHE PATENTSCHRIFT

(11) **EP 1 425 384 B1**
(45) Veröffentlichungstag und Bekanntmachung des Hinweises auf die Patenterteilung: **20.12.2006**
(21) Anmeldenummer: 02776674.0
(22) Anmeldetag: 13.09.2002
(51) Int. Cl.: C12N 1/00, C12M 1/26, C12M 1/20, C12M 1/34

(54) **VERFAHREN ZUR KULTIVIERUNG UND ANALYSE MIKROBIELLER EINZELZELLKULTUREN**
METHODS FOR CULTIVATING AND ANALYZING MICROBIAL INDIVIDUAL CELL CULTURES
PROCEDE POUR CULTIVER ET ANALYSER DES CULTURES DE CELLULES INDIVIDUELLES MICROBIENNES

(30) Priorität: 14.09.2001 DE 10145568
(43) Veröffentlichungstag der Anmeldung: 09.06.2004
(73) Patentinhaber: HANS-KNÖLL-INSTITUT FÜR NATURSTOFF-FORSCHUNG E.V., 07745 Jena (DE); INSTITUT FÜR BIOPROZESS- UND ANALYSENMESSTECHNIK E.V., 37308 Heiligenstadt (DE); INSTITUT FÜR PHYSIKALISCHE HOCHTECHNOLOGIE E.V., 07745 Jena (DE)
(72) Erfinder: MÜLLER, Peter-Jürgen, 07745 Jena (DE); ROTH, Martin, 07749 Jena (DE); GROTH, Ingrid, 07751 Wogau Stadt Jena (DE); KUMMER, Christel, 07751 Cospeda (DE); MARTIN, Karin, 07751 Grosslöbichau (DE); SCHROECKH, Volker, 07751 Rothenstein (DE); METZE, Josef, 37308 Heiligenstadt (DE); KÖHLER, Michael, 07751 Golmsdorf (DE); HENKEL, Thomas, 07745 Jena (DE); GASTROCK, Gunter, 37308 Heiligenstadt (DE); LEMKE, Karen, 37085 Göttingen (DE); HILLIGER, Matthias, verstorben (DE)
(74) Vertreter: Bock, Gerhard
(86) Internationale Anmeldenummer: PCT/DE2002/003451
(87) Internationale Veröffentlichungsnummer: WO 2003/025113

(56) Entgegenhaltungen:
- WO-A-82/02561
- DE-A- 3 915 920
- MANOME AKIRA ET AL: "Application of gel microdroplet and flow cytometry techniques to selective enrichment of non-growing bacterial cells." FEMS MICROBIOLOGY LETTERS, Bd. 197, Nr. 1, 2001, Seiten 29-33, XP002233815 ISSN: 0378-1097 in der Anmeldung erwähnt

## Beschreibung

Mit der Erfindung kann eine der am häufigsten durchgeführten Grundoperationen der Mikrobiologie, das Vereinzeln und die parallele Kultivierung der Einzelorganismen mit einer bisher nicht erreichten großen Anzahl von mikrobiellen Individuen durchgeführt werden.

Ziel der Vereinzelung ist beispielsweise das Auffinden von Mikroorganismen mit herausragenden Eigenschaften. Mikroorganismen mit herausragenden Eigenschaften treten in Mikroorganismenkulturen und -populationen häufig in einer sehr kleinen Zahl, gemessen an der Gesamtzahl, auf. Sie entstehen ständig in jeder mikrobiellen Population durch spontane Mutation, sie werden gezielt durch Mutagenese, durch Transfektion, Transformation und durch gentechnische Methoden künstlich erzeugt oder kommen als Kontamination in die Kultur.

Die Suche (Screening) nach neuen Mikroorganismen mit neuen, besseren Eigenschaften aber auch der Nachweis von Mikroorganismen mit pathogenen oder schädlichen Eigenschaften erfolgt in Proben, die als Suspensionen an natürlichen oder anthropomorph beeinflußten Standorten oder Produkten, beispielsweise Böden oder Nahrungsmittel, und in wäßrigen Habitaten, beispielsweise Abwassereinrichtungen, oder aus lebenden höheren Organismen gewonnen werden.

Mit der Erfindung wird ein Weg eröffnet, um mikrobielle Einzelorganismen bzw. mikrobielle Individuen mit neuen bzw. besonderen Leistungen oder Eigenschaften in einer vielzahligen, in Bezug auf die Mikroorganismen homogen oder heterogen zusammengesetzten Populationen aufzufinden und damit das große Potential der mikrobiellen Leistungen besser nutzen zu können.

Gleichfalls ist die Erfindung mit Vorteil einzusetzen, wenn die Lebenstätigkeit von Mikroorganismen als Indikator zur qualitativen oder quantitativen Bestimmung von Nährsubstraten oder Effektoren des Wachstums und des Stoffwechsels, z. B. von Antibiotika oder von essentiellen Nährstoffkomponenten, eingesetzt wird. Auch die Optimierung von Nährstoffzusammensetzungen der zur Kultivierung verwendeten Medien ist möglich.

Es entsteht die Möglichkeit, diese Untersuchungen mit seiner sehr hohen Zahl von monoklonalen Zellen bzw. Kulturen in Form von Mikro-Reinkulturen durchzuführen.

Die Erfindung kann überall dort genutzt werden, wo mikrobielle Leistungen oder Wirkungen gesucht, angewendet, verbessert oder analysiert werden, beispielsweise in der Biotechnologie, der Gentechnik, der medizinischen Mikrobiologie, der Pharmazie, der Mikrobiologie und der Lebensmittel- und Umweltmikrobiologie.

Das Herstellen von Reinkulturen bzw. monoklonalen Kulturen bzw. nicht kontaminierten Kulturen ist eine Grundoperation in der Mikrobiologie [1]. Eine Reinkultur besteht aus den Nachkommen einer einzigen Zelle. Ihre Zellen haben gleiche Wachstums- und Stoffwechseleigenschaften. Zur Gewinnung von Reinkulturen ist die Isolierung von Einzelzellen für die Beimpfung der Kulturen notwendig.

Das Auffinden und Gewinnen der interessanten Individuen aus einer aus sehr vielen Mikroorganismen zusammengesetzten Submerskultur (Flüssigkultur) bzw. einer Zellsuspension stellt aber ein bisher nicht befriedigend gelöstes wissenschaftlichtechnisches Problem dar [2]. Bisher wurden diese Arbeiten nur mit unzulänglichen Methoden bzw. durch Plattieren stark verdünnter Kultursuspensionen auf Agaroberflächen durchgeführt, oder es wurden durch mechanische Manipulation Einzelzellen isoliert, beispielsweise mit einer sogenannten Laserpipette [3]. Die Anwendung von Methoden der mechanischen Zellsortierung ist beim jetzigen Stand der Technik wegen der großen Anzahl zu trennender Organismen noch sehr zeitaufwendig bzw. praktisch nicht durchführbar.

Für die parallele Durchführung von großzahligen Kultivierungen könnten theoretisch sehr kleine Kultivierungsgefäße eingesetzt werden. Die prinzipielle Verwendung mikrotechnisch hergestellter Kavitäten und Kanäle für biotechnologische Kultivierungen wurde bereits vorgeschlagen [4]. Erste Versuche zum praktischen Einsatz der Mikrosystemtechnik zeigten, dass die Paralielbeimpfung und Kultivierung von 16 Mikrokulturen (*Escherichia coli*) im 0.1-µl-Maßstab möglich ist [5]. Eine Pipettierung von Mikroorganismen ist mit Portionierern realisierbar [6].

Eine Vereinzelung der zur Beimpfung eingesetzten Mikroorganismen aus einer großzahligen, z. B. 10⁵ bis 10⁸ Zellen umfassenden Population wird bisher wegen der dann erheblich größeren Anzahl von erforderlichen Mikrokulturgefäßen nicht durchgeführt.

Mikroorganismen werden für eine Reihe von biotechnischen Anwendungen in Gelkügelchen (gel microdroplets; GMD) eingeschlossen. Es wird eine Mikroorganismen und ein wasserlösliches, gelbildendes Material enthaltende Suspension mit Hilfe eines speziellen Düsensystems in möglichst kleine Tropfen, die einzelne Zellen enthalten, aufgeteilt und anschließend zu GMDs verfestigt oder mikroverkapselt [7-9]. Zur Kultivierung der Mikroorganismen werden die GMDs in einem flüssigen Nährmedium inkubiert. Das Wachstum und ausgewählte Eigenschaften der Mikroorganismen in den einzelnen GMPs können mit verschiedenen Methoden detektiert werden [7-9]. Nachteilig an dieser Methode ist, dass schnell wachsende Mikroorganismen bereits nach kurzer Kultivierungszeit aus den GMDs in die umgebende Nährlösung austreten können und dadurch alle anderen GMDs kontaminiert werden. Insbesondere zur Kultivierung, Charakterisierung und Isolierung unterschiedlich schnell wachsender Mikroorganismen oder Zellen ist diese Methode deshalb nicht geeignet. Ein weiterer Nachteil besteht darin, dass mehrfache Messungen an den einzelnen GMPs und die zugehörige Datenerfassung technisch kaum realisierbar sind.

Beim Isolieren von Mutanten, Selektanten, Kontaminanten oder von gentechnisch veränderten Mikroorganismen werden fast ausschließlich klassische Methoden angewendet. Die Zellpopulationen werden soweit verdünnt, dass nach Auftragen der verdünnten Bakteriensuspensionen auf die Oberfläche von Agarnährböden separate Kolonien bzw. Einzelkolonien entstehen, die jeweils aus einer Mikroorganismenzelle hervorgegangen sind.

Zusätzlich werden häufig selektive Bedingungen durch gezieltes Substratangebot oder Zusatz von Wachstumseffektoren erzeugt, bei denen nur die angestrebten Mikroorganismen wachsen können.

Bei gentechnischen Arbeiten werden die Gene, die übertragen werden sollen, mit Markergenen gekoppelt. Es handelt sich bei den Markergenen häufig um Resistenzgene gegen Antibiotika. Dadurch wachsen in Kulturen, die mit Antibiotikum versetzt wurden, nur die Klone, die das übertragene Gen enthalten.

In vielen Fällen existiert keine einfache Möglichkeit, die meist in geringer Zahl vorhandenen interessanten Mikroorganismen auf einfachem und direktem Weg zu erkennen und zu gewinnen, d. h. zu isolieren.

Exemplarisch soll der Stand der Technik an der Vorgehensweise bei einem Primärscreening, der Suche nach neuen Mikroorganismen mit neuen Leistungen, dargestellt werden [10]. Hochrechnungen von Zählungen in Extrakten aus Bodenproben zeigten, dass maximal nur etwa 1 bis 10 % der mit einer durchschnittlichen Anzahl von insgesamt etwa 10⁶ bis 10⁸ pro g Bodenprobe vorkommenden Mikroorganismen gleicher und unterschiedlicher Arten mit dieser traditionellen Suchtechnik aufgefunden werden.

Die Proben werden beim traditionellen Primärscreening in einem Puffer oder Wasser suspendiert, um definierte mikrobielle Suspensionen (Submersproben) zu erhalten. Die festen Begleitstoffe z. B. Erde werden abgetrennt und der flüssige, die abgespülten Mikroorganismen enthaltende Überstand (Extrakt) so lange verdünnt (Verdünnungsschritte), bis bei nachfolgendem Auftragen auf Agaroberflächen emers wachsende, durch wachstumsfreie Zonen voneinander getrennte bzw. isolierte mikrobielle Einzelkolonien auftreten. Diese werden isoliert und auf interessierende Leistungen und Eigenschaften geprüft. Das vorrangige Ziel der Verdünnung ist es, separate bzw. nicht kontaminierte Kolonien (Reinkulturen) zu erhalten.

Bei einer typischen Vorgehensweise wird im Primärscreening 1 g einer Erdprobe (berechnet als Trockengewicht) mit 10 ml eines Puffers, einer Salzlösung oder Wasser suspendiert und bei gut besiedelten Gartenböden durch Verdünnungsschritte bis etwa 10⁶-fach verdünnt. Mit der Verdünnungstufe, bei der Einzelkolonien auftreten, werden Petrischalen mit Agarmedien mit je 0,1 ml Extraktverdünnung beimpft. Diese Vorgehensweise führt dazu, dass nur die Mikroorganismen, die nach der Verdünnung noch in 0,1 ml Extraktverdünnung anwesend sind, auf der Agaroberfläche anwachsen können.

Nach der Inkubation kann auf der Basis der gewachsenen Koloniezahlen und der angewandten Verdünnungsschritte die Anwesenheit von beispielsweise 10⁶ bis 10⁸ koloniebildenden Einheiten je g Bodentrockenmasse berechnet werden. Die Kultivierung dieser großen Zahl von Mikroorganismen ist jedoch mit der angewandten Methode nicht möglich. Aus der Vielzahl der gewachsenen Mikroorganismenkolonien wird anschließend meist unter Berücksichtigung morphologischer Merkmale von ausgewählten Kolonien überimpft und weiter kultiviert. Die ausgewählten Klone werden anschließend in einem Sekundärscreening auf neue Stoffwechselleistungen untersucht. Mikroorganismenspezies, die z. B. in einer 100mal geringeren Konzentration in der Bodenprobe vorhanden sind, werden mit einer 100fach geringeren Wahrscheinlichkeit mit der beschriebenen Vorgehensweise gefunden.

Neben dem durch das Verdünnungsregime bedingten Verlust an Mikroorganismen im Probenmaterial gibt es weitere Gründe, die einem kompletten Auffinden entgegenstehen. Sie sind in der Physiologie der Mikroorganismen zu suchen.

Etwa 90 % der in der Bodenprobe vorhandenen Mikroorganismen werden pauschal zu den "nicht kultivierbaren" Mikroorganismen gerechnet. Nicht kultivierbar bedeutet, dass diese Mikroorganismen unter den gewählten Wachstumsbedingungen nicht wachsen. Um sie zu kultivieren, müssen die Wachstumsbedingungen den speziellen Anforderungen der Mikroorganismen an Nährstoffe und physikalische Parameter angepaßt werden. Es besteht das Problem, dass sich ein Teil der Mikroorganismen in ihrem Biotop/Ökosystem in einem physiologisch inaktiven Zustand (Dormanz) befinden (K-Strategen). Sie sind lebensfähig, aber unter den üblicherweise verwendeten Standardbedingungen sowie den angewendeten Kultivierungszeiten nicht kultivierbar. Andere Mikroorganismen (r-Strategen) wachsen sehr schnell. Ein Grund für das Nichtfinden eines Großteils der Mikroorganismen könnte darin bestehen, dass die K-Strategen bzw. die "nicht kultivierbaren" unter den Mikroorganismen häufig nicht anwachsen bzw. durch r-Strategen überwachsen werden.

Es gibt inzwischen Hinweise, dass das Wachstum von Mikroorganismen durch Wachstumsfaktoren reguliert wird. Es ist ein bekanntes Phänomen, dass hin und wieder zu dünn beimpfte Kulturen nicht anwachsen. Gibt man zu der beimpften Kultur eine geringe Menge des Filtrates aus einer wachsenden Kultur eines Mikroorganismus, wird das Wachstum induziert.

Es ist das Ziel der Erfindung, die mikrobiologischen Grundoperationen der Zellvereinzelung und Einzelzellkultivierung in großer Zahl auf einfachem Weg parallel durchzuführen. Damit soll der mikrobiellen Spezifik Rechnung getragen werden, dass Reinkulturen für viele Anwendungen eigentlich erforderlich wären, aber wegen der hohen Individuenzahlen in den Kulturen bisher nicht auf einfachem Weg realisiert werden können. Dies führte beispielsweise bisher dazu, dass in einer Mikroorganismenpopulation in sehr geringer Zahl vorhandene Arten mit interessanten oder herausragenden Eigenschaften nicht gefunden werden. Mikrobielle Submerskulturen sollen deshalb so behandelt werden, dass möglichst jeder der in einer Zellsuspension vorhandenen Mikroorganismen die Möglichkeit erhält, als Einzelorganismus in einer separaten Kultivierungssphäre als Reinkultur bzw. Mikrokultur zu wachsen. Es soll damit auch verhindert werden, dass bei einem Screening durch die traditionell notwendigerweise angewendeten Verdünnungsschritte die praktisch isolierbare Individuenzahl in großem Maß vermindert wird.

Um Mikroorganismen mit auffälligen Eigenschaften oder mikrobielle Infektionen oder neue bzw. seltene Mikroorganismen, darunter die K-Strategen sowie die nicht oder schwer kultivierbaren Mikroorganismen, zu erkennen, zu finden und zu isolieren oder um die Wirkung von Effektoren an Hand einer großen Zahl paralleler Wachstumsversuche statistisch auswertbar zu untersuchen, stellt sich die erfinderische Aufgabe, Verfahren zu entwickeln, mit denen alle Mikroorganismen einer wässrigen Mikroorganismensuspension, die eine hohe Zahl von gleichartigen oder unterschiedlichen Mikroorganismen enthält, in Form von Reinkulturen kultiviert werden können.

Diese Aufgabe beinhaltet die Entwicklung einer Methode zur Vereinzelung aller in einer Kultur vorhandenen Mikroorganismen durch die Nutzung der durch die Mikrosystemtechnik gegebenen Möglichkeiten. Weiterhin sollen gegebenenfalls die Wachstumsbedingungen so variiert werden können, dass vereinzelten Mikroorganismen mit ausgewählten Eigenschaften ein Wachstum ermöglicht wird, andere, nicht erwünschte jedoch nicht oder nur begrenzt wachsen können.

Zur Lösung dieser Aufgaben wird erfindungsgemäß ein Verfahren zur Parallelkultivierung von Mikroorganismen vorgeschlagen, dass dadurch gekennzeichnet ist, dass einer von groben Feststoffen befreiten Suspension oder Kultur einer homogen oder heterogen zusammengesetzten Mikroorganismenpopulation Nährsubstrate und/oder Effektoren und/oder mikrobielle Metabolite zugesetzt werden, anschließend ein Volumen v der mikrobiellen Suspension, welche N Mikroorganismen enthält, mit einem Portionierer in n₁ Teilvolumina geteilt wird, wobei die Zahl n₁ zwischen N und 100N, bevorzugt zwischen N und 10N gewählt wird, anschließend die Teilvolmina gegebenenfalls unter Zuführung von Nährsubstraten und/oder Effektoren zur Beimpfung von n₂ separaten Mikrokulturen in Mikroarealen oder Mikrokavitäten, wobei n₂ größer/gleich n₁ ist, verwendet werden, anschließend die Mikrokulturen inkubiert und während des Wachstums gegebenfalls weitere Nährsubstrate und/oder Effektoren und/oder Metabolite zugeführt und physiologische Parameter und das Wachstum der einzelnen Mikrokulturen mit geeigneten Messverfahren erfasst werden.

Mikroorganismen im Sinne dieser Erfindung sind prokaryontische und eukaryontische Zellen, wobei die Zellen einzeln und/oder als Zellverbände/Zellaggregate und/oder als Gewebsfragmente vorliegen können. Zu den prokaryontischen Zellen zählen Bakterien und Blaualgen, die eukaryontischen Zellen umfassen Hefen, Pilze, tierische und pflanzliche Zellen.

Die Zahl N/v wird beispielsweise mikroskopisch durch Zählen in einer Bakterien- oder Blutzählkammer oder durch andere an sich bekannte Methoden ermittelt. Die Zahl n₁ liegt erfindungsgemäß zwischen 10⁴ und 10⁸.

Das Volumen der bei der Vereinzelung entstehenden Teilvolumina liegt zwischen 0,1 nl und 1 µl, die sie aufnehmenden Mikrokavitäten und die Mikrokulturen haben ein Volumen von 0,1 nl bis 10 µl.

Alle für den Aufbau der Mikroorganismenzellen essentiellen Elemente (C, O, H, N, S, P, K, Na, Ca, Mg, Fe) und sogenannte Spurenelemente werden in für die Zellen verfügbarer Form als Nährsubstrate zugeführt.

Darüber hinaus werden den Mikrokulturen erfindungsgemäß Effektoren des mikrobiellen Wachstums zugesetzt wie z. B. Wachstumsaktivatoren oder Wachstumsinhibitoren, Ezyminhibitoren oder Enzymaktivatoren, Antibiotika, Cytokine, Enzyme, Vitamine, Aminosäuren, Antimetabolite sowie mikrobielle Metabolite.

Durch die gezielte Zugabe von Effektoren des mikrobiellen Wachstums kann das Wachstum unerwünschter Mikroorganismen unterdrückt bzw. das Wachstum von gewünschten Mikroorganismen gefördert werden, oder es werden bestimmte Produktbildungen bzw. Stoffwechselleistungen der Mikrokulturen induziert.

Effektoren des mikrobiellen Wachstums beeinflussen das Wachstum positiv oder negativ. Die Zugabe antibiotischer Substanzen entsprechend der Art der Reinkultur und in Abhängigkeit von ihrer Konzentration führt zur Wachstumsinhibition nichtresistenter Mikroorganismen. Beispielsweise verhindert der Zusatz von antifungalen Antibiotika das Wachstum von Pilzen, die die für das erfindungsgemäße Vorgehen sehr nachteilige Eigenschaft aufweisen, bakterielle Mikrokulturen zu überwachsen. Wenn Bakterien kultiviert werden sollen, werden deshalb antifungal wirkende Substanzen zugegeben, um das Wachstum störender Pilze zu verhindern.

Der Zusatz von Antimetaboliten inhibiert das Wachstum über eine negative Beeinflussung von Stoffwechselwegen. Außerdem können bei einer weiteren Art der Kultivierung ein oder mehrerer Antibiotika in hoher Konzentration zugesetzt werden, die nur auf wachsende Mikroorganismen wirken und diese hemmen (z. B. ein Penicillinderivat). Anschließend wird die Kultur zentrifugiert und die Antibiotika werden mit dem Überstand entfernt.

Wachstumsfördernde Metabolite werden in reiner Form oder in Kulturfiltraten prokaryontischer und/oder eukaryontischer Zellkulturen und/oder in Konzentraten derselben und/oder in Extrakten aus prokaryontischen und/oder eukaryontischen Zellkulturen zugesetzt. Dadurch wird beispielsweise das Wachstum von schwer kultivierbaren Mikroorganismen stimuliert.

Die technische Realisierung der Mikrokulturen erfolgt erfindungsgemäß in Mikrokavitäten. Erfindungsadäquate Methoden sind Entfernung von Feststoffen, Vereinzeln, Portionieren, Beimpfen, Nährstoffversorgung einschließlich Sauerstoffversorgung und Zuführung von mikrobiellen Metaboliten und Effektoren des mikrobiellen Wachstums, Herstellung selektiver Wachstumsbedingungen und Messen von Wachstum und Produktbildung. Der Vorgang der Vereinzelung ist vorzugsweise technisch mit dem Vorgang der Mikrokultivierung eng verbunden. Die an sich bekannten Bedingungen für mikrobielle Kultivierungen wie Konstanthaltung physiologisch verträglicher Temperaturen und der Azidität werden mit an sich bekannten Methoden eingehalten. Die Sterilität der Vorrichtungen wird in an sich bekannter Weise durch Erhitzen mit Dampf auf 121 °C, durch trockenes Erhitzen auf Temperaturen über 150 °C, durch chemische Sterilisation oder Sterilisation mittels Strahlung erreicht.

Die Details der erfindungsgemäßen Vorgehensweise werden im folgenden erläutert.

Es wird z. B. eine Bodenprobe mit einem einfachen Puffer oder Wasser versetzt und nach gründlichem Schütteln durch Zentrifugieren die Feststoffe sedimentiert, diese entfernt und anschließend die Mikroorganismen durch Zentrifugation als Pellet gewonnen. Das Pellet wird in einem Medium, das alle essentiellen Nährsubstrate sowie gegebenfalls Effektoren des mikrobiellen Wachstums enthält, suspendiert.

Unerwünschte, schnellwachsende Bakterien werden abgetötet, indem ein oder mehrere Antibiotika zugesetzt werden, die nur auf wachsende Mikroorganismen wirken (z. B. Penicillin). Nach beispielsweise 4 h Inkubation wird die Kultur zentrifugiert und die Antibiotika im Überstand werden entfernt.

Die Mikrokultivierungen erfolgen erfindungsgemäß in Mikrokavitäten, die mit den durch Vereinzelung erhaltenen Teilvolumina vollständig oder teilweise gefüllt werden. Die erfindungsgemäße Vorgehensweise weist durch Nutzung der Möglichkeiten der Mikrosystemtechnik einen vorteilhaften neuartigen Weg zu einer systemgerechten Behandlung der bei mikrobiellen Problemstellungen in der Regel besonders hohen Individuenzahlen.

Die erfindungsgemäß eingesetzten Mikrokavitäten sind in der Regel zweidimensional angeordnet. Das Volumen der Mikrokavitäten liegt zwischen 0,1 nl und 10 µl.

Die Vereinzelung der in der Suspension vorhandenen Mikroorganismen oder ein Vereinzeln der Mikroorganismen wird über die Befüllung von Mikrokavitäten in Form von Mikrokapillaren bzw. in einem Array angeordneten Mikrokapillaren mit einem Volumenäquivalent zwischen 0,1 nl und 1 µl realisiert.

Die Kultivierung der vereinzelten Mikroorganismen erfolgt bei diesem Verfahren in Mikrokapillaren in voneinander separierten Mikrokulturen mit einem Volumenzwischen 0,1 nl und 1 µl.

Zur Vereinzelung der Mikroorganismen werden insbesondere ein miniaturisierter thermisch gesteuerter Fluidschalter oder ein miniaturisierter Fluidschalter in Verbindung mit einer Mikroinjektionseinheit oder ein pneumatisch getriebener Fluidschalte verwendet.

Alternativ wird ein auf elektrischen Wirkprinzipien beruhender Schalter verwendet, der als elektrostatischer oder elektromagnetischer oder dielektrophoretischer Schalter ausgeführt ist.

Durch periodisches Wechseln der Volumenflußrate durch Gasblasen oder durch mit Wasser nicht mischbare Trennflüssigkeiten werden Flüssigkeitssegmente erhalten, die mit einer Wahrscheinlichkeit < 5 % mehr als eine Zelle pro Segment enthalten. Eine Einzelkapillare wird mit einer Vielzahl solcher Flüssigkeitssegmente befüllt und enthält die beschriebene Vielzahl von separierten Einzelkompartimenten mit je einer Zelle.

Die Durchmischung bzw. der Sauerstoffübergang über das offene Ende der Kapillaren wird durch pulsierende Druckschwankungen in den Kapillaren verbessert.

Die Mikrokultivierung kann erfindungsgemäß auch in einer Vielzahl von Kapillaren erfolgen, wobei jede Kapillare eine Mikrokavität darstellt. Die Befüllung mit Kulturflüssigkeit bzw. der Beimpfungsprozeß erfolgt aktiv durch Zuführung oder passiv durch Ansaugen über die Kapillarkräfte. Durch pulsierende Druckänderung an einem Ende der Kapillare wird eine Hin- und Herbewegung der Kulturflüssigkeit in der Kapillare erzeugt und damit die Durchmischung bzw. der Sauerstoffübergang über das offene Ende der Kapillare verbessert.

Durch erfindungsgemäße Nutzung eines Fluidsystems mit serieller Probenauftrennung wird eine eindimensionale Mikrokulturvariante eingesetzt. Die aus der Fließinjektionsanalyse bekannten technischen Anordnungen und Systeme werden hierbei für die mikrobielle Kultivierung benutzt. Durch parallele Mehrfachanordnung wird die Zahl der Mikrokulturen erhöht.

In Chips eingebrachte Mikrokapillaren dienen als Speicher- und Kulturräume. In einem einzigen Chip von etwas mehr als 2 cm² Fläche ist eine Kapillarlänge von etwa 1 m untergebracht. Mit einer Sperrflüssigkeit werden die Mikrokulturen in den Kapillaren voneinander getrennt. In der Kapillare von 1 m Länge werden etwa 5000 Proben mit einem Einzelvolumen von etwa 0,1 nl kultiviert. 200 dieser Chips werden eingesetzt, um etwa 1 Million Mikrokulturen aufzunehmen. Das Gesamtvolumen dieser Chips ist kleiner als 100 ml. Bei einer signifikanten Erhöhung der Einzelvolumina um den Faktor 10 (auf etwa 1 nl) liegt das Gesamtvolumen aller Chips bei einem Liter.

Für die eindimensionale Kultivierung von Proben, deren Gesamtvolumen größer als 1 Liter ist, werden Schleifen von preisgünstig erhältlichem Schlauchmaterial für die durch kleine Fluidabschnitte segmentierte Speicherung der Proben verwendet.

Vorteilhaft an der erfindungsgemäßen Vereinzelung der Mikroorganismen enthaltenen Suspensionen oder Kulturen in die Volumenäquivalente ist, dass durch die Aufteilung jedes Volumenäquivalent im Durchschnitt einen individuellen Mikroorganismus enthält. Jeder der vereinzelten Mikroorganismen kann entsprechend seines Wachstumsverhaltens sehr schnell oder erst nach einer längeren Verzögerungsphase anwachsen, ohne dass die langsam wachsenden Individuen von schneller wachsenden Individuen überwachsen werden.

In einer bestimmten begrenzten Anzahl von Fällen, besonders dann wenn n₁ = N ist, enthalten sie einen, mehr als einen Mikroorganismus oder keinen. Die leeren Äquivalente können auf Grund des fehlenden Wachstums erkannt werden.

Zur Systemsteuerung wird zum Zeitpunkt der Mikroorganismen-Vereinzelung und deren Einbringung in eine Mikrokavität oder ein Mikroarreal die jeweilige Koordinatenzuordnung auf einem Festspeichermedium abgelegt und registriert, womit eine eindeutige Zuordnung zu jedem Zeitpunkt möglich ist.

Zum Vereinzeln der Mikroorganismen wird ein System von Portionierern benutzt, bei dem das Volumen der einzeln abgegebenen Tropfen zwischen 0,1 nl und 1 µl liegt und jeweils 1 Tropfen in ein Mikroarreal oder eine Mikrokavität abgegeben wird.

Dabei werden die Tropfen mittels einer Volumenimpulsoptimierung ohne Bildung von Spritzern abgegeben.

In einer anderen Ausführungsform wird zum Vereinzeln der Mikroorganismen ein Portionierer, dessen Zuführung mit einer Partikel- bzw. Zellzähleinrichtung versehen ist, benutzt, der die Mikroorganismen enthaltende Flüssigkeit in Einzeltropfen von 0,1 nl bis 1 µl Volumen abgibt und die Befüllung einer Aufnahmeposition abbricht, wenn entweder deren maximales Füllvolumen erreicht oder ein eine Zelle enthaltender Tropfen abgesetzt wurde.

Alternativ wird zum Vereinzeln der Mikroorganismen ein piezoelektrisch gesteuerter Portionierer eingesetzt, wobei die Tropfenfrequenz und die Tropfengröße der Vorschubbewegung der Positioniereinrichtung sowie der Zellkonzentration, dem Innenvolumen und der Ortsfrequenz der Probenaufnahmebereiche so angepaßt werden, dass mit einer Wahrscheinlichkeit <5% mehr als eine Zelle pro Aufnahmeposition abgegeben wird.

Als eine weitere Variante zum Vereinzeln der Mikroorganismen wird ein pneumatisch oder elektropneumatisch gesteuerter Portionierer eingesetzt, wobei die Tropfenfrequenz und die Tropfengröße der Vorschubbewegung der Positioniereinrichtung sowie der Zellkonzentration, dem Innenvolumen und der Ortsfrequenz der Probenaufnahmebereiche so angepaßt werden, dass mit einer Wahrscheinlichkeit < 5 % mehr als eine Zelle pro Aufnahmeposition abgegeben wird.

In einer weiteren erfindungsgemäßen Ausführungsform werden für die kompartimentierte Kultivierung von Mikroorganismen Nanotiterplatten [11] mit Kavitäten im Volumenbereich von 0,01 bis 500 nl pro Kavität eingesetzt.

Nach dem Vereinzeln erfolgt die Kultivierung der Mikrokulturen in Mikrokavitäten, die in einem Array mit einem Abstand zueinander von gleich oder kleiner als 1,8 mm angeordnet sind.

Hierfür sind insbesondere Nanotiterplatten mit Mikrokavitäten, die eine konische oder eine zylinderförmige oder eine kugelsegmentförmige oder eine prismatische, pyramidale, doppel- oder mehrfach-pyramidale Form besitzen, geeignet.

Nach dem Vereinzeln werden die Mikrokulturen in den Kammern von Nanotiterplatten kultiviert.

Die Gas- und Nährstoffversorgung der Mikrokulturen kann durch eine Mikroporenmembran erfolgen, deren Porenweite vorzugsweise zwischen 0.1 µm und 4 µm und deren Membrandicke zwischen 0,2 µm und 10 µm liegt, so dass die Zellen zurückgehalten werden.

Zu diesem Zweck kann die Nährstoffversorgung in den Mikrokulturen durch eine Mikroporenmembran oder durch eine Nanoporenmembran erfolgen, die versorgungsseitig durch ein Mikrofluidkanalsystem abgedeckt ist.

Bei bestimmten Ausführungsformen erhalten die Mikrokavitäten der Nanotiterplaten über die Mikro- oder Nanoporenmembranen eine gemeinsame Versorgung, während Effektoren des mikrobiellen Wachstums optional von oben in die Mikrokavitäten appliziert werden.

Ebenso kann die Versorgung der Mikrokulturen durch eine Mikroporenmembran mit einem oder mehreren Mikrokanälen erfolgen, die dergestalt in eine (Mikro-)Fließ-Injektionsanordnung eingebunden sind, dass die Wirkung von Effektoren oder Nährsubstraten durch Injektion in den Perfusionskanal einfach und seriell getestet werden können.

Die Herstellung der zur Versorgung dienenden Mikrofluidkanäle, welche eine Mikroporenmembran tragen, wird durch eine Folge von einem isotropen und einem anisotropen Ätzschritt in Silizium realisiert.

Zur Verbesserung der Handhabbarkeit und zur Vermeidung des fluidischen Übersprechens während der Befüllung können die Stege zwischen den Mikrokammern mit einer wasserabweisenden Oberflächenschicht versehen werden.

Voraussetzung für die Selektion von Mikroorganismen mit bestimmten Eigenschaften ist der analytische Zugang zu physiologischen und Kulturparametern. Erfindungsgemäß ist der vollständige oder teilweise Einsatz der nachfolgend benannten Methoden und konstruktiven Merkmale vorgesehen.

Zur Analyse physiologischer Parameter und zur Messung des Wachstums in den jeweiligen Mikrokulturen wird vorzugsweise die Methode der **E**lektro**i**mpedanz**s**pektroskopie (EIS) eingesetzt.

Die Kinetik der Kulturparameter pH, pO₂, pCO₂, wird mittels spektroskopischer Verfahren vor und nach dem Anströmen der diffusiven Versorgung der in Suspension vorhandenen Mikroorganismen detektiert. Alternativ wird das Wachstum der Mikrokulturen mikroturbidometrisch oder photometrisch verfolgt.

Zur Messung des Wachstums der Mikrokulturen werden Chipkammern mit mindestens 2 zueinander parallel und in sich planparallel angeordneten transparenten Seitenwänden verwendet.

Diese zueinander planparallelen Seitenwände sind optional partiell mit einer hochreflektierenden Dünnschicht ausgestattet, wobei in diese mikrostrukturierte Fenster zum Ein- und Auskoppeln des Lichtes eingebracht sind.

Das Wachstum der Mikrokulturen wird durch die Erhöhung des Strömungswiderstandes beim Bewegen der kleinen Flüssigkeitsvolumina aufgrund der zunehmenden Gesamtviskosität der die Zellen enthaltenden Flüssigkeit verfolgt.

Alternativ wird Wachstum der Mikrokulturen durch die Verstärkung der Ablenkung, Fokussierung oder Defokussierung eines nichtabsorbierten Laserstrahles beim Aufheizen der die Zellen enthaltenden Flüssigkeit durch einen durch die Zellen teilweise absorbierten Laserstrahl verfolgt.

Zur Detektion der Strahlposition des Meßlichtes werden Empfänger-Doppelzeilen eingesetzt und mit deren Hilfe die den einzelnen Positionen entsprechenden Differenzen der Asymmetrien der Lichtintensitäten in den lokalen Kulturbereichen als Meßgrößen verwendet.

### Ausführungsbeispiel

Ein Nährmedium mit 2 g Hefeextrakt, 20 g Malzextrakt und 10 g Glucose pro Liter wird mit Hefenzellen der Art *Saccharomyces cerevisiae* beimpft. Nach 18 h Inkubation bei 30 °C als Standkultur wird die Zahl der in der Kultur befindlichen Hefezellen unter Verwendung einer mikroskopischen Zählkammer durch Auszählen mit Hilfe eines Mikroskops ermittelt. Anschließend wird die Suspension so verdünnt und auf einem Agarmedium (2 g Hefeextrakt, 20 g Malzextrakt und 15 g Agar pro Liter, pH 6,2) in 10 cm-Petrischalen plattiert, dass ca. 25 Zellen pro cm² aufgetragen werden. Die Petrischalen werden 3 h bei 30 °C inkubiert.

Zur kompartimentierten Klonierung werden Kavitäten von Nanotiterplatten mit flüssigem Agarmedium (2 g Hefeextrakt, 20 g Malzextrakt, 6 g Agar, pH 6,2) gefüllt und mit formschlüssigen Silikonsternpeln gedeckelt. Nach dem Aushärten des Agars werden jeweils die Silikonstempel entfernt und durch einen zweiten Silikonstempel ersetzt, auf den vorher Zellen von den vorkultivierten Agarplatten durch Stempeln übertragen wurden. Vor dem Stempeln werden die vorkultivierten Agarplatten 20 min bei 37 °C getrocknet und der Silikonstempel auf 37 °C temperiert. Der beimpfte Silikonstempel wird mittels einer Klemmvorrichtung so an die Nanotiterplatte angedrückt, dass die Stege der Nanotiterplatte durch den Silikonstempel abgedichtet werden. Die so beimpften Nanotierplatten werden bei 30°C inkubiert. Das Wachstum in den Kavitäten der Nanotiterplatten wird mittels Trübungsmessung unter Verwendung eines Auflichtmikroskops verfolgt. Die Entnahme von Klonen zur weiteren Kultivierung und Prüfung erfolgt mittels einer sterilen Impfnadel unter Zerstörung der am Boden der Nanotiterplatte befindlichen Membran.

Für die Kultivierung werden Nanotiterplatten aus Silizium mit metallverstärkter Bodenmembran verwendet. Die Kammeröffnung beträgt 800 x 800 µm bei einem Raster von 1 mm. Die Bodenweite beträgt ca. 150 x 150 µm, das gesamte Kammervolumen etwa 150 nl. (Hersteller: Institut für Physikalische Hochtechnologie e.V. Jena, Abt. Biotechnische Mikrosysteme, Winzerlaer Straße 10, 07745 Jena, http://www.ipht-jena.de). Silikonstempel werden durch Abformen von Nanotiterplatten mit identischer Geometrie und auf 100 µm verringerter Ätztiefe hergestellt. Als Abformmaterial wird handelsübliches additionsvernetzendes Silikon eingesetzt (Hersteller z. B. Sylgard).

### Literaturzitate

[1] Chan, E. C. S., Pelczar, M. J., Krieg, N. R. (1993) Verwendung von Reinkulturen - Grundvorausetzung für Studium der Mikroorganismen. In: Laboratory Exercise in Microbiology. Chan, E. C. S., Pelczar, M. J., Krieg, N (eds.). McCraw-Hill, New York, 123-152
[2] Gottschal, J. C., Harder, W., Prins, R.A. (1992) In: The Procaryotes. Balows A., Trüper, H. G., Dworkin, M., Harder, W., Schleifer K.-H. (eds.). Springer, New York 149-196
[3] Huber, R. (1999) Die Laserpipette als Basis für Einzelkultivierungen. Biospektrum 4, 289-291
[4] Kroy, W., Seidel, H., Dette, E., Deimel, P., Binder, F., Hilpert, R., Königer, M. (1990) DE 3915920
[5] Schober, A., Schlingloff, G., Thamm, A., Vetter, D., Thomandl, D., Gebinoga, M., Kiel, H. J., Scheffler, C., Döhring, M., Köhler, J. M., Mayer, G. (1996) Systemintegration of Microsystems/Chip Elements in miniaturized automats for high-throughput synthesis and screening in biology, biochemistry and chemistry. Micro System Technologies (Potsdam Okt. 1996), Proc. S. 705, Vortrag
[6] Schober, A., Günther, R., Schwienhorst, A., Döring, M., Lindemann, B.F. (1993) BioTechniques 15, 324
[7] Weaver, J. C. (1981) US-Patent 4401755
[8] Williams, G. B., Weaver, J. C., Demain, A. L. (1990) Rapid microbial detection and enumeration using gel microdroplets and colorimetric or fluorescence indicator systems. J. Clin. Microbiol. 28, 1002-1008
[9] Manome, A., Zhang, H., Tani, Y., Katsuragi, T., Kurane, R., Tsuchida, T. (2001) Application of gel microdroplet and flow cytometry techniques to selective enrichmant of non-growing cells. FEMS Microbiol. Lett. 197, 29-33
[10] Omura, S. (1986) Philosophy of new drug discovery. Microbiol. Reviews 50, 259-279
[11] Mayer, G., Wohlfart, K., Schober, A., Köhler, J. M. (1999) Nanotiterplates for Synthesis and Screening. In: Microsystem technology: a powerful tool for biomolecular studies. Köhler, J. M., Mejevaia, T., Saluz, H. P. (eds.). p. 75-128, Birkhäuser Basel

## Patentansprüche

1. Verfahren zur Parallelkultivierung von Mikroorganismen, **dadurch gekennzeichnet, dass** einer von groben Feststoffen befreiten Suspension oder Kultur einer homogen oder heterogen zusammengesetzten Mikroorganismenpopulation Nährsubstrate und/oder Effektoren und/oder mikrobielle Metabolite zugesetzt werden, anschließend ein Volumen v der mikrobiellen Suspension, welche N Mikroorganismen enthält, mit einem Portionierer in n₁ Teilvolumina geteilt wird, wobei die Zahl n₁ zwischen N und 100N, bevorzugt zwischen N und 10N gewählt wird, anschließend die Teilvolmina gegebenenfalls unter Zuführung von Nährsubstraten und/oder Effektoren zur Beimpfung von n₂ separaten Mikrokulturen in Mikroarealen oder Mikrokavitäten, wobei n₂ größer/gleich n₁ ist, verwendet werden, anschließend die Mikrokulturen inkubiert und während des Wachstums gegebenfalls weitere Nährsubstrate und/oder Effektoren und/oder Metabolite zugeführt und physiologische Parameter und das Wachstum der einzelnen Mikrokulturen mit geeigneten Messverfahren erfasst werden.

2. Verfahren nach Anspruch 1, **dadurch gekennzeichnet, dass** als Mikroorganismen prokaryontische und eukaryontische Zellen definiert werden.

3. Verfahren nach den Ansprüchen 1 und 2, **dadurch gekennzeichnet, dass** gleichvolumige Teilvolumina erzeugt werden und die Zahl n₁ zwischen 10⁴ und 10⁸ liegt.

4. Verfahren nach den Ansprüchen 1 bis 3, **dadurch gekennzeichnet, dass** das Volumen der bei der Vereinzelung entstehenden Teilvolumina zwischen 0,1 nl und 1 µl, das Volumen der sie aufnehmenden Mikrokavitäten und das Volumen der resultierenden Mikrokulturen zwischen 1 nl und 10 µl liegt.

5. Verfahren nach den Ansprüchen 1 bis 4, **dadurch gekennzeichnet, dass** den Mikrokulturen wachstumsfördemde Metabolite enthaltende Kulturfiltrate prokaryontischer und/oder eukaryontischer Zellkulturen und/oder Konzentrate derselben und/oder Extrakte aus prokaryontischen und/oder eukaryontischen Zellkulturen zugesetzt werden.

6. Verfahren nach den Ansprüchen 1 bis 5, **dadurch gekennzeichnet, dass** den Mikrokulturen Effektoren wie Wachstumsaktivatoren oder Wachstumsinhibitoren, Ezyminhibitoren oder Enzymaktivatoren, Wirkstoffe, Antibiotika, Cytokine, Vitamine, Aminosäuren, Enzyme oder Antimetabolite zugesetzt werden.

7. Verfahren nach den Ansprüchen 1 bis 6, **dadurch gekennzeichnet, dass** die Vereinzelung der in der Suspension vorhandenen Mikroorganismen oder ein Vereinzeln der Mikroorganismen über die Befüllung von Mikrokavitäten in Form von Mikrokapillaren bzw. in einem Array angeordneten Mikrokapillaren mit Teilvolumina der resultierenen Mikrokulturen zwischen 0,1 nl und 1 µl erfolgt.

8. Verfahren nach Anspruch 7, **dadurch gekennzeichnet, dass** die Kultivierung der vereinzelten Mikroorganismen in Mikrokapillaren in voneinander separierten Mikrokulturen mit einem Volumen zwischen 0,1 nl und 1 µl erfolgt.

9. Verfahren nach Anspruch 7, **dadurch gekennzeichnet, dass** zum Vereinzeln der Mikroorganismen ein miniaturisierter thermisch gesteuerter Fluidschalter verwendet wird.

10. Verfahren nach den Ansprüchen 7 und 8, **dadurch gekennzeichnet, dass** zum Vereinzeln der Mikroorganismen ein miniaturisierter Fluidschalter in Verbindung mit einer Mikroinjektionseinheit oder ein pneumatisch getriebener Fluidschalter verwendet wird.

11. Verfahren nach Ansprüchen 7 und 8, **dadurch gekennzeichnet, dass** zum Vereinzeln von Mikroorganismen ein auf elektrischen Wirkprinzipien beruhender Schalter verwendet wird, der als elektrostatischer oder elektromagnetischer oder dielektrophoretischer Schalter ausgeführt ist.

12. Verfahren nach den Ansprüchen 7 und 8, **dadurch gekennzeichnet, dass** in einem geschlossenen Mikrokapillarsystem durch periodisches Wechseln der Volumenflußrate durch Gasblasen oder durch mit Wasser nicht mischbare Trennflüssigkeiten Flüssigkeitssegmente erzeugt werden, die mit einer Wahrscheinlichkeit < 5 % mehr als eine Zelle pro Segment enthalten.

13. Verfahren nach den Ansprüchen 7 und 8, **dadurch gekennzeichnet, dass** die Durchmischung bzw. der Sauerstoffübergang über das offene Ende der Kapillaren durch pulsierende Druckschwankungen in den Kapillaren verbessert wird.

14. Verfahren nach den Ansprüchen 1 bis 6, **dadurch gekennzeichnet, dass** nach dem Vereinzeln die Kultivierung der Mikrokulturen in Mikrokavitäten erfolgt, die in einem Array mit einem Abstand zueinander von gleich oder kleiner als 1,8 mm angeordnet sind.

15. Verfahren nach Anspruch 14, **dadurch gekennzeichnet, dass** die Mikrokavitäten eine konische oder eine zylinderförmige oder eine kugelsegmentförmige oder eine prismatische, pyramidale, doppel- oder mehrfach-pyramidale Form besitzen.

16. Verfahren nach Anspruch 14, **dadurch gekennzeichnet, dass** nach dem Vereinzeln die Mikrokulturen in den Kammern von Nanotiterplatten kultiviert werden.

17. Verfahren nach Anspruch 14, **dadurch gekennzeichnet, dass** die Nährstoffversorgung der Mikrokulturen durch eine Mikroporenmembran erfolgt, deren Porenweite vorzugsweise zwischen 0,1 µm und 4 µm und deren Membrandicke zwischen 0,2 µm und 10 µm liegt, so dass die Zellen zurückgehalten werden.

18. Verfahren nach Anspruch 14, **dadurch gekennzeichnet, dass** die Nährstoffversorgung in den Mikrokulturen durch eine Mikroporenmembran oder durch eine Nanoporenmembran erfolgt, die versorgungsseitig durch ein Mikrofluidkanalsystem abgedeckt ist.

19. Verfahren nach Anspruch 14, **dadurch gekennzeichnet, dass** alle Mikrokavitäten der Nanotiterplaten über die Mikro- oder Nanoporenmembranen eine gemeinsame Versorgung erhalten und Effektoren des Wachstums von oben in die Mikrokavitäten appliziert werden.

20. Verfahren nach Anspruch 14, **dadurch gekennzeichnet, dass** die Versorgung der Mikrokulturen durch eine Mikroporenmembran unter Verwendung eines oder mehrerer Mikrokanäle erfolgt, die dergestalt in eine (Mikro-)Fließ-Injektionsanordnung eingebunden sind, dass die Wirkung von Effektoren oder Nährsubstraten durch Injektion in den Perfusionskanal einfach und seriell getestet werden kann.

21. Verfahren nach Anspruch 14, **dadurch gekennzeichnet, dass** die zur Versorgung dienenden Mikrofluidkanäle eine Mikroporenmembran tragen, die durch eine Folge von einem isotropen und einem anisotropen Ätzschritt in Silizium hergestellt werden.

22. Verfahren nach Anspruch 14, **dadurch gekennzeichnet, dass** die Stege zwischen den Mikrokammern mit einer wasserabweisenden Oberflächenschicht versehen sind.

23. Verfahren nach den Ansprüchen 1 bis 6, **dadurch gekennzeichnet, dass** zur Analyse physiologischer Parameter und zur Messung des Wachstums in den jeweiligen Mikrokulturen die Methode der **E**lektro**i**mpedanz**s**pektroskopie (EIS) eingesetzt wird.

24. Verfahren nach den Ansprüchen 1 bis 6, **dadurch gekennzeichnet, dass** die Kinetik der Kulturparameter pH, pO₂, pCO₂, mittels spektroskopischer Verfahren vor und nach dem Anströmen der diffusiven Versorgung der in Suspension vorhandenen Mikroorganismen detektiert wird.

25. Verfahren nach den Ansprüchen 1 und 6, **dadurch gekennzeichnet, dass** das Wachstum der Mikrokulturen mikroturbidometrisch oder photometrisch verfolgt wird.

26. Verfahren nach den Ansprüchen 1 und 6, **dadurch gekennzeichnet, dass** zur Messung des Wachstums der Mikrokulturen Chipkammern mit mindestens 2 zueinander parallel und in sich planparallel angeordneten transparenten Seitenwänden verwendet werden.

27. Verfahren nach Anspruch 26, **dadurch gekennzeichnet, dass** die zueinander planparallelen Seitenwände partiell durch eine hochreflektierende Dünnschicht ausgestattet sind, wobei in diese mikrostrukturierte Fenster zum Ein- und Auskoppeln des Lichtes eingebracht sind.

28. Verfahren nach den Ansprüchen 1 bis 6, **dadurch gekennzeichnet, dass** das Wachstum der Mikrokulturen durch die Erhöhung des Strömungswiderstandes beim Bewegen der kleinen Flüssigkeitsvolumina aufgrund der zunehmenden Gesamtviskosität der die Zellen enthaltenden Flüssigkeit verfolgt wird.

29. Verfahren nach den Ansprüchen 1 bis 6, **dadurch gekennzeichnet, dass** das Wachstum der Mikrokulturen durch die Verstärkung der Ablenkung, Fokussierung oder Defokussierung eines nichtabsorbierten Laserstrahles beim Aufheizen der die Zellen enthaltenden Flüssigkeit durch einen durch die Zellen teilweise absorbierten Laserstrahl verfolgt wird.

30. Verfahren nach den Ansprüchen 1 bis 6, **dadurch gekennzeichnet, dass** zur Detektion der Strahlposition des Meßlichtes Empfänger-Doppelzeilen eingesetzt und mit deren Hilfe die den einzelnen Positionen entsprechenden Differenzen der Asymmetrien der Lichtintensitäten in den lokalen Kulturbereichen als Meßgrößen verwendet werden.

31. Verfahren nach den Ansprüchen 1 bis 6, **dadurch gekennzeichnet, dass** zur Systemsteuerung zum Zeitpunkt der Mikroorganismen-Vereinzelung und deren Einbringung in eine Mikrokavität oder ein Mikroarreal die jeweilige Koordinatenzuordnung auf einem Festspeichermedium abgelegt und registriert wird, womit eine eindeutige Zuordnung zu jedem Zeitpunkt möglich wird.

32. Verfahren nach den Ansprüchen 1 bis 6, **dadurch gekennzeichnet, dass** zum Vereinzeln der Mikroorganismen ein System von Portionierern benutzt wird, bei dem das Volumen der einzeln abgegebenen Tropfen zwischen 0,1 nl und 1 µl liegt und jeweils 1 Tropfen in ein Mikroarreal oder eine Mikrokavität abgegeben wird.

33. Verfahren nach den Ansprüchen 1 bis 6, **dadurch gekennzeichnet, dass** die Tropfen mittels einer Volumenimpulsoptimierung ohne Bildung von Spritzern abgegeben werden.

34. Verfahren nach den Ansprüchen 1 bis 6, **dadurch gekennzeichnet, dass** zum Vereinzeln der Mikroorganismen ein Portionierer, dessen Zuführung mit einer Partikel- bzw. Zellzähleinrichtung versehen ist, benutzt wird, der die Mikroorganismen enthaltende Flüssigkeit in Einzeltropfen von 0,1 nl bis 1 µl Volumen abgibt und die Befüllung einer Aufnahmeposition abbricht, wenn entweder deren maximales Füllvolumen erreicht oder ein eine Zelle enthaltender Tropfen abgesetzt wurde.

35. Verfahren nach den Ansprüchen 1 bis 6, **dadurch gekennzeichnet, dass** zum Vereinzeln der Mikroorganismen ein piezoelektrisch gesteuerter Portionierer eingesetzt wird, wobei die Tropfenfrequenz und die Tropfengröße der Vorschubbewegung der Positioniereinrichtung sowie der Zellkonzentration, dem Innenvolumen und der Ortsfrequenz der Probenaufnahmebereiche so angepaßt werden, dass mit einer Wahrscheinlichkeit <5% mehr als eine Zelle pro Aufnahmeposition abgegeben wird.

36. Verfahren nach den Ansprüchen 1 bis 6, **dadurch gekennzeichnet, dass** zum Vereinzeln der Mikroorganismen ein pneumatisch oder elektropneumatisch gesteuerter Portionierer eingesetzt wird, wobei die Tropfenfrequenz und die Tropfengröße der Vorschubbewegung der Positioniereinrichtung sowie der Zellkonzentration, dem Innenvolumen und der Ortsfrequenz der Probenaufnahmebereiche so angepaßt werden, dass mit einer Wahrscheinlichkeit < 5 % mehr als eine Zelle pro Aufnahmeposition abgegeben wird.

## Claims

1. Procedure for the parallel cultivation of microorganisms wherein nutrient substrates and/or effectors and/or microbial metabolites are added to a suspension or culture of a homogeneously or heterogeneously composed microorganism population being freed of rough solids, and afterwards, a volume v of the microbial suspension, which contains N microorganisms, is divided into n1 partial volumes by using a proportional device, with the amount of n1 being selected between N and 100N, preferably between N and 10N, then, the partial volumes are used, if required by adding nutrient substrates and/or effectors, for injecting n2 separate microcultures in microareas or microcavities, with n2 being bigger /equal n1, and now the microcultures are incubed and during the growth further nutrient substrates and/or effectors and/or metabolites are added if required and the physiological parameters and the growth of the individual microcultures are registered by using appropriate measurement procedures.

2. Procedure as set forth in claim 1, wherein procaryontic and eucaryontic cells are defined as microorganisms.

3. Claims as set forth in claims 1 and 2, wherein partial volumes of the same volume are generated and the number n1 amounts to a value of between 10⁴ and 10⁸.

4. Procedure as set forth in claims 1 through 3, wherein the volume of the partial volumes generated by isolation amounts to a value between 0.1 nl and 1 µl and the microcavities and microcultures taking them up have a volume of between 1 nl and 10 µl.

5. Procedure as set forth in claims 1 through 4, wherein culture filtrates of procaryontic and/or eucaryontic cell cultures and/or concentrates of them and/or extracts of procaryontic and/or eucaryontic cell cultures containing growth-supporting metabolites are added to the microcultures.

6. Procedure as set forth in claims 1 through 5, wherein effectors such as growth activators or growth inhibitors, enzyme inhibitors or enzyme activators, active substances, antibiotics, cytocines, vitamins, amino acids, enzymes or antimetabolites are added to the microcultures.

7. Procedure as set forth in claims 1 through 6, wherein the isolation of the microorganisms existing in the suspension or the isolation of the microorganisms is realized by filling a partial volume of the resulting microcultures of between 0.1 nl and 1 µl into microcavities that are designed as microcapillaries or as microcapillaries arranged in an array.

8. Procedure as set forth in claim 7, wherein the cultivation of the isolated microorganisms is performed in microcapillaries in microcultures that are separated from each other and have a volume of between 0.1 nl and 1 µl.

9. Procedure as set forth in claim 7, wherein a miniaturized thermically controlled fluid switch is used for the isolation of the microorganisms.

10. Procedure as set forth in claims 7 and 8, wherein a miniaturized fluid switch combined with an injection micro-unit or a pneumatically driven fluid switch is used for the isolation of the microorganisms.

11. Procedure as set forth in claims 7 and 8, wherein a switch based on electrical principles is used for the isolation of microorganisms and said switch can be an electrostatic or an electromagnetic one.

12. Procedure as set forth in claims 7 and 8, wherein in a closed microcapillary system the periodic change of the volume flow rate by means of gas bubbles or by means of separation liquids that do not mix with water produce liquid segments that contain more than one cell per segment with a probability of < 5 %.

13. Procedure as set forth in claims 7 and 8, wherein the thorough mixing or the oxygen transfer via the open end of the capillary is improved by pulsating pressure variations in the capillaries.

14. Procedure as set forth in claims 1 through 6, wherein after the isolation, the cultivation of the microcultures starts in the microcavities that are arranged in an array and at a distance of maximally 1.8 mm to each other.

15. Procedure as set forth in claim 14, wherein the microcavities are designed as cylinders or spherical segments or prisms, pyramids, or double- or multiple pyramids.

16. Procedure as set forth in claim 14, wherein after the isolation, the microcultures are cultivated in the chambers of the nanotiter plates.

17. Procedure as set forth in claim 14, wherein nutrients are supplied to the microcultures via a micropore membrane with a preferential pore width of between 0.1 µm and 4 µm and a membrane thickness of between 0.2 µm and 10 µm so that the cells are kept back.

18. Procedure as set forth in claim 14, wherein the nutrients are supplied to the microcultures through a micropore membrane or through a nanopore membrane that is covered by a microfluid channel system at the supplying side.

19. Procedure as set forth in claim 14, wherein all microcavities of the nanotiter plates obtain a common supply via the micro- or nanopore membranes and growth effectors are added into the microcavities from the top.

20. Procedure as set forth in claim 14, wherein the supply of the microcultures is performed through a micropore membrane provided with one or several microchannels that so belong to a (micro)flow injection arrangement so that the effect of effectors or nutrient substances can be easily tested in series by means of injection into the perfusion channel.

21. Procedure as set forth in claim 14, wherein the microfluid channels that are used for supplying purposes and are provided with a micropore membrane are manufactured in a sequence of an isotropic and an anisotropic etching process in silicon.

22. Procedure as set forth in claim 14, wherein a hydrophobic surface film is applied onto the surface of the bridges located between the microchambers.

23. Procedure as set forth in claims 1 through 6, wherein electro-impedance spectroscopy (EIS) is used for analyzing the physiological parameters and for measuring the growth in the corresponding microcultures.

24. Procedure as set forth in claims 1 through 6, wherein the kinetic conditions of the culture parameters pH, pO2, pCO2 are detected by means of spectroscopic procedures before and after the flow of the diffusive supply for the microorganisms existing in the suspension.

25. Procedure as set forth in claims 1 through 6, wherein the growth of the microcultures is controlled by means of micro-turbidometric or photometric methods.

26. Procedure as set forth in claims 1 through 6, wherein chip chambers are used for measuring the growth of the microcultures and said chambers are provided with at least 2 transparent lateral walls that are arranged parallel to each other and are plane-parallel.

27. Procedure as set forth in claim 26, wherein said lateral walls in plane-parallel arrangement to each other are partially covered with a highly-reflective thin layer that is provided with microstructured windows for coupling in and out the light.

28. Procedure as set forth in claims 1 through 6, wherein the growth of the microcultures is monitored on the basis of the increase of the flow resistance during the movement of the small liquid volumes with said resistance increase being caused by the increase of the total viscosity of the cell-containing liquid.

29. Procedure as set forth in claims 1 through 6, wherein the growth of the microcultures is observed via a laser beam partly absorbed by the cells in such a way that the deviation, focusing or defocusing of a non-absorbed laser beam is intensified when the cell-containing liquid is heated.

30. Procedure as set forth in claims 1 through 6, wherein receiver double-cells are used to detect the beam position of the measuring light, and with their help the differences of the asymmetries of the light intensities in the local culture zones are taken as measuring values.

31. Procedure as set forth in claims 1 through 6, wherein for system control purposes, the appropriate coordinate assignment is stored and registered on a ROM medium at the time of the isolation of the microorganisms and their injection into a microcavity or a microarea thus allowing a clear assignment at any point in time.

32. Procedure as set forth in claims 1 through 6, wherein a system of proportioning devices is used to isolate the microorganisms and in said proportioning device the volume of the individually released droplets amounts to between 0.1 nl and 1 µl and always one droplet is taken by an microarea or one microcavity.

33. Procedure as set forth in claims 1 through 6, wherein the droplets are released via a volume impulse optimization system without the formation of splashes.

34. Procedure as set forth in claims 1 through 6, wherein a proportioning device the supply of which is provided with a particle or counter unit is used for isolating the microorganisms and said proportioning device releases the microorganisms-containing liquid in single droplets with a volume ranging from 0.1 nl to 1 µl and stops the filling, if the maximum filling volume has been reached or a droplet containing a cell has been released.

35. Procedure as set forth in claims 1 through 6, wherein a proportioning device controlled by piezoelectricity is used for the isolation of the microorganisms and wit h said proportioning device the droplet frequency and size are adapted to the feed motion of the positioning unit and the cell concentration, the interior volume and the local frequency of the sample-uptaking areas in such a way that, with a probability of < 5 %, more than one cell is released per uptaking position.

36. Procedure as set forth in claims 1 through 6, wherein a pneumatically or electro-pneumatically proportioning device is used for the isolation of the microorganisms and with said proportioning device the droplet frequency and size are adapted to the feed motion of the positioning unit and the cell concentration, the interior volume and the local frequency of the sample-uptaking areas in such a way that, with a probability of < 5 %, more than one cell is released per uptaking position.

## Revendications

1. Procédé de culture simultanée de micro-organismes est **caractérisé en ce que** des substrats nutritifs et/ou des effecteurs et/ou des métabolites microbiens sont ajoutés à une suspension ou une culture exempte de particules solides grossières d'une population de micro-organismes de composition hétérogène, que, par la suite, un volume v de la suspension microbienne comprenant N micro-organismes est subdivisé à l'aide d'un portionneur en n₁ volumes partiels, n₁ allant de N à 100 N , de préférence de N à 10 N, que, ensuite, les volumes partiels, le cas échéant après addition de substrats nutritifs et/ou d'effecteurs sont utilisés pour inoculer n₂ microcultures séparées dans des microzones de distribution ou microcavités, n₂ étant supérieur ou égal à n₁, que, ensuite, les microcultures sont incubées, et que, pendant leur croissance, sont additionnés éventuellement d'autres substrats nutritifs et/ou effecteurs et/ou métabolites, et que la croissances des différentes microcultures ainsi que des paramètres physiologiques seront saisis par des procédés de mesures appropriées.

2. Procédé selon la revendication 1 est **caractérisé en ce que** les cellules procaryotes et eukaryotes sont considérées comme micro-organismes.

3. Procédé selon les revendications 1 et 2 est **caractérisé en ce que** des volumes partiels et égaux sont isolés et que le chiffre n₁ se situe entre 10⁴ et 10⁸.

4. Procède selon les revendications 1 à 3 est **caractérisé en ce que** le volume créés lors de l'individualisation des volumes partiels se situe entre 0,1nl et 1µl et que le volume des microcavités qui les absorbe et le volume des microcultures qui en résultent, se situent entre 1 nl à 10 µl.

5. Procédé selon les revendications 1 à 4 est **caractérisé en ce qu'**aux microcultures sont additionnés des filtrats de cultures de cellules procaryotes et/ou de cellules eukaryotes et/ou des concentrés de celles-ci et/ou des extraits de cultures de cellules procaryotes et/ou de cellules eukaryotes, contenant tous des métabolites favorisant la croissance.

6. Procédé selon les revendications 1 à 5 est **caractérisé en ce qu'**aux microcultures sont additionnées des effecteurs tels que activateurs et inhibiteurs de croissance, inhibiteurs ou activateurs d'enzymes, substances actives, antibiotiques, cytokines, vitamines, acides aminés, enzymes ou antimétabolites.

7. Procédé selon les revendications 1 à 6 est **caractérisé en ce que** l'individualisation des micro-organismes contenus dans la suspension ou l'individualisation des micro-organismes est obtenue par l'intermédiaire du remplissage de microcavités en forme de microcapillaires ou de microcapillaires disposés en array présentant des portions de microcultures d'un volume respectif allant de 0,1 nl à 1 µl.

8. Procédé selon la revendication 7 est **caractérisé en ce que** la culture de micro-organismes individualisés dans les microcapillaires est obtenue dans des microcultures séparées les unes des autres, d'un volume allant de 0,1 nl à 1 µl.

9. Procédé selon la revendication 7 est **caractérisé en ce qu'**on utilise un switch pour fluides miniaturisés à commande thermique pour l'individualisation des micro-organismes.

10. Procédé selon les revendications 7 et 8 est **caractérisé en ce que**, pour l'individualisation des micro-organismes, on utilise soit un switch pour fluides miniaturisés en rapport avec une micro-unité d'injection soit un switch pour fluides à commande pneumatique.

11. Procédé selon les revendications 7 et 8 est **caractérisé en ce qu'**on utilise, pour l'individualisation de micro-organismes, un switch fonctionnant à base de principes d'action électriques et conçu comme un switch électrostatique, électromagnétique ou diélectrophorétique.

12. Procédé selon les revendications 7 et 8 sont **caractérisés en ce que**, dans un système capillaire fermé, sont produits, en alternant périodiquement les débits des volumes à l'aide de bulles de gaz ou des liquides séparateurs non miscibles à l'eau, des segments de liquide comprenant plus qu'une cellule par segment avec une probabilité de < 5%.

13. Le Procédé selon les revendications 7 et 8 est **caractérisé en ce que** le mélange ou le cas échéant le passage de l'oxygène à travers l'extrémité ouverte des capillaires sera amélioré grâce aux variations de pression pulsées dans les capillaires.

14. Procédé selon les revendications 1 à 6 est **caractérisé en ce que**, après l'individualisation la culture des microcultures se fait dans les microcavités disposées dans un microarray à une distance les unes des autres égale ou inférieure à 1,8 mm.

15. Procédé selon la revendication 14 est **caractérisé en ce que** les microcavités ont une forme conique ou cylindrique ou encore la forme d'un segment sphérique ou bien celle d'un prisme ou d'une pyramide, double ou multiple.

16. Procédé selon la revendication 14 est **caractérisé en ce que**, après l'individualisation, les microcultures sont cultivées dans des compartiments de nano-plaques de titrage.

17. Procédé selon la revendication 14 est **caractérisé en ce que** l'alimentation en substances nutritives des microcultures a lieu a travers une micromembrane aux pores écartées les unes des autres de préférence de 0,1 µm à 4 µm, l'épaisseur de la membrane allant de 0,2µm à 10 µm, ce qui permet, de cette façon, de retenir les cellules.

18. Procédé selon la revendication 14 est **caractérisé en ce que** l'alimentation en substances nutritives des microcultures a lieu a travers une micromembrane ou une nanomembrane et que, côté alimentation, un système de microcanaux assure l'arrivée des fluides.

19. Procédé selon la revendication 14 est **caractérisé en ce que** toutes les microcavités des nano-plaques de titrage sont alimentées à travers des membranes aux micro- ou nano-pores par un seul système d'alimentation et que des effecteurs de croissance sont appliqués par le haut dans les microcavités.

20. Procédé selon la revendication 14 est **caractérisé en ce que** l'alimentation des microcultures est assurée a travers une membrane à micro-pores et l'utilisation d'une ou plusieurs microcanaux, qui, de cette façon, sont intégrées dans une (micro) configuration d'écoulement et d'injection de sorte que l'effet des effecteurs ou des substrats nutritifs peut être testé de manière simple ou en série, par injection dans le canal à perfusion

21. Procédé selon la revendication 14 est **caractérisé en ce que** les microcanaux à fluide assurant l'alimentation sont dotés d'une membrane dont les micro-pores sont obtenus par une gravure anisotrope de silicium.

22. Procédé selon la revendication 14 est **caractérisé en ce que** les ponts entre les microcompartiments sont dotés à leur surface d'une couche hydrophobe.

23. Procédé selon les revendications 1 à 6 est **caractérisé en ce que** pour l'analyse des paramètres physiologiques et pour la mesure de la croissance dans les microcultures respectives, c'est la méthode de la **s**pectroscopie d'**i**mpédance **é**lectrique (SIE) qui est appliquée.

24. Procédé selon les revendications 1 à 6 est **caractérisé en ce que** la cinétique des paramètres des cultures pH, pO₂, pCO₂ est détectée au moyen de procédés spectroscopiques avant et après l'apport de l'alimentation diffuse des micro-organismes en suspension.

25. Procédé selon les revendications 1 et 6 est **caractérisé en ce que** la croissance des microcultures est suivie de façon microturbidométrique ou photométrique.

26. Procédé selon les revendications 1 et 6 est **caractérisé en ce que** des compartiments aux puces comprenant au moins deux parois latérales transparentes aux faces disposées en parallèle l'une par rapport à l'autres, ou aux faces planes et parallèles, sont utilisés pour mesurer la croissance des microcultures.

27. Procédé suivant la revendication 26 est **caractérisé en ce que** les parois latérales aux faces planes et parallèles les unes par rapport aux autres, sont partiellement dotées d'une couche mince à haute réflexion dans laquelle des fenêtres microstructurées sont intégrées pour permettre de laisser passer la lumière ou non.

28. Procédé selon les revendications 1 à 6 est **caractérisé en ce que** la croissance des microcultures est suivie au moyen de l'augmentation de la résistance à l'écoulement à cause de la croissance de la viscosité totale du liquide contenant des cellules.

29. Procédé selon les revendications 1 à 6 est **caractérisé en ce que** la croissance des microcultures moyennant le renforcement de la déviation, la focalisation ou défocalisation d'un faisceau laser non absorbé pendant l'échauffement du liquide contenant les cellules, peut être suivie par un faisceau laser absorbé en partie par les cellules.

30. Procédé selon les revendications 1 à 6 est **caractérisé en ce que** des récepteurs double ligne sont appliqués pour la détection des positions du faisceau de mesure, avec l'aide desquels les écarts dans les asymétries des intensités lumineuses dans les zones locales des cultures correspondant aux différentes positions sont utilisés comme grandeurs de mesure.

31. Procédé selon les revendications 1 à 6 est **caractérisé en ce que** l'attribution aux coordonnées est déposée et enregistrée sur un support de mémoire fixe ce qui permet à tout moment une attribution précise dans l'intérêt de la gestion des systèmes au moment de l'individualisation des micro-organismes et de leur intégration dans une microcavité ou un microarray.

32. Procédé selon les revendications 1 à 6 est **caractérisé en ce que** pour l'individualisation des micro-organismes, tout un système de portionneurs est utilisé, **caractérisé par le fait que** le volume des gouttes sortant individuellement est de 0,1 nl à 1µl et que les gouttes soient toujours évacuées l'une après l'autres vers une micro-aire ou une microcavité.

33. Procédé selon les revendications 1 à 6 est **caractérisé en ce que** les gouttes sont évacuées par optimisation des impulsions des volumes sans produire de projections.

34. Procédé selon les revendications 1 à 6 est **caractérisé en ce que** pour l'individualisation des micro-organisme un portionneur est utilisé dont le système d'alimentation est doté d'un compteur de particules ou de cellules et qui évacue par gouttes individuelles de 0,1 nl à 1µl de volume le liquide contenu dans les micro-organismes et arrête le remplissage d'une position de réception au moment où le volume de remplissage maxi est atteint ou une goutte contenant une cellule a été évacuée.

35. Procédé selon les revendications 1 à 6 est **caractérisé en ce que** pour l'individualisation des micro-organismes est utilisé un portionneur à commande piézo-électrique et que la fréquence et la dimension des gouttes sont adaptées au mouvement d'avancement du système de positionnement, à la concentration des cellules, au volume intérieur et à la fréquence du site des zones de réception d'échantillons de sorte qu'avec une probabilité de plus de < 5%, plus d'une cellule par position de réception est évacuée.

36. Procédé selon les revendications 1 à 6 est **caractérisé en ce que** pour l'individualisation des micro-organismes est utilisé un portionneur à commande pneumatique ou électropneumatique et que la fréquence et la dimension des gouttes sont adaptées au mouvement d'avancement du système de positionnement, à la concentration des cellules, au volume intérieur et à la fréquence au site des zones de réception d'échantillons de sorte qu'avec une probabilité de < 5%, plus d'une cellule par position de réception est évacuée.
